# EUROPÄISCHE PATENTSCHRIFT

(11) **EP 2 563 333 B1**
(45) Veröffentlichungstag und Bekanntmachung des Hinweises auf die Patenterteilung: **26.07.2017**
(21) Anmeldenummer: 11738394.3
(22) Anmeldetag: 02.05.2011
(51) Int. Cl.: A61K 36/52, A61K 36/28, A61K 36/288, A61K 36/11, A61K 36/49, A61K 36/185, A61K 9/08, A61K 9/12, A61K 9/00, A61K 9/19

(54) **AUSGEWÄHLTE PFLANZENEXTRAKTE ZUR BEHANDLUNG VON ENTZÜNDUNGSERKRANKUNGEN**
SELECTED PLANT EXTRACTS FOR THE TREATMENT OF INFLAMMATORY DISEASES
EXTRAITS VÉGÉTAUX SÉLECTIONNÉS POUR LE TRAITEMENT DE MALADIES INFLAMMATOIRES

(30) Priorität: 30.04.2010 DE 102010019099
(43) Veröffentlichungstag der Anmeldung: 06.03.2013
(73) Patentinhaber: Bionorica SE, 92318 Neumarkt (DE)
(72) Erfinder: BAUER, Rudolf, 8042 Graz (AT); KOPEINIG, Birgit, 8010 Graz (AT); POPP, Michael, 91207 Lauf-Heuchling (DE)
(74) Vertreter: Simandi, Claus
(86) Internationale Anmeldenummer: PCT/EP2011/002173
(87) Internationale Veröffentlichungsnummer: WO 2011/134679

(56) Entgegenhaltungen:
- WO-A1-2011/048221
- WO-A2-2010/049542
- BIONORICA AG: "Imupret bringt das Immunsystem auf Trab", INTERNET CITATION, [Online] Seiten 1-2, XP002570480, Gefunden im Internet: URL:http://www.news-ticker.ch/pm.php?news_ id=4762461&aktion=nf> [gefunden am 2010-02-25]
- PAHL A: "Imupret modulates the innate and adaptive immune system parameters in vitro", PLANTA MEDICA, Bd. 75, Nr. 9, Juli 2009 (2009-07), Seite 1080, XP009152087, & 57TH INTERNATIONAL CONGRESS AND ANNUAL MEETING OF THE SOCIETY-FOR-MEDICINAL-PLANT-RESEARCH-AND-N ATUR; GENEVA, SWITZERLAND; AUGUST 16 -20, 2009 ISSN: 0032-0943
- AMMON H P T ET AL: "KAMILLE MECHANISMUS DER ANTIPHLOGISTISCHEN WIRKUNG VON KAMILLENEXTRAKTEN UND -INHALTSSTOFFEN//CHAMOMILE. MECHANISM OF ANTIPHLOGISTIC ACTIVITY OF CHAMOMILE EXTRACTS AND THEIR CONTENTS", DEUTSCHE APOTHEKER ZEITUNG, DEUTSCHER APOTHEKER VERLAG, STUTTGART, DE, Bd. 136, Nr. 22, 1. Januar 1996 (1996-01-01), Seiten 17/18,21-24,27, XP008039864, ISSN: 0011-9857
- DO MONTE FABRICIO HOFFMANN MARTINS ET AL: "Antinociceptive and anti-inflammatory properties of the hydroalcoholic extract of stems from Equisetum arvense L. in mice", PHARMACOLOGICAL RESEARCH, ACADEMIC PRESS, LONDON, GB, Bd. 49, Nr. 3, 1. März 2004 (2004-03-01), Seiten 239-243, XP009126388, ISSN: 1043-6618, DOI: 10.1016/J.PHRS.2003.10.002 [gefunden am 2003-12-02]
- KATAOKA MASAHIRO ET AL: "Effect of the 50 percent MeOH extracts of crude drug (standards of natural drugs not in the J.P. XII) on beta-hexosaminidase release from rat basophilic leukemia (RBL-2H3) cells", NATURAL MEDICINES, Bd. 50, Nr. 5, 1996, Seiten 344-348, XP009153787, ISSN: 1340-3443
- QA'DAN FADI ET AL: "The antimicrobial activities of Psidium guajava and Juglans regia leaf extracts to acne-developing organisms", AMERICAN JOURNAL OF CHINESE MEDICINE, Bd. 33, Nr. 2, 2005, Seiten 197-204, XP009153786, ISSN: 0192-415X
- NURGUN ERDEMOGLU ET AL: "Anti-inflammatory and antinociceptive activity assessment of plants used as remedy in Turkish folk medicine", JOURNAL OF ETHNOPHARMACOLOGY, Bd. 89, Nr. 1, 1. November 2003 (2003-11-01), Seiten 123-129, XP55011983, ISSN: 0378-8741, DOI: 10.1016/S0378-8741(03)00282-4

## Beschreibung

Die Erfindung offenbart Pflanzenextrakte aus wenigstens einem Extrakt aus wenigstens einem Pflanzenmaterial ausgewählt aus *Equiseti herba* (Schachtelhalmkraut), *Juglandis folium* (Walnussblätter), *Millefolii herba* (Schafgarbenkraut), *Quercus cortex* (Eichenrinde), *Taraxaci herba* (Löwenzahnkraut), *Althaeae radix* (Eibischwurzel) und *Matricariae flos* (bzw. *Flos chamomillae* (Kamilleblüten)) oder eine Mischung oder Unterkombination davon. Ferner betrifft die Erfindung ein daraus erhältliches antientzündliches Mittel und Arzneimittel.

Imupret® (eingetragene Marke der BIONORICA SE) ist eine bekannte Mischung aus Pflanzendrogen, die aus diesen Pflanzenmaterialien bereit gestellt wird. Durch eine Kombination dieser sieben Heilpflanzen erhält man eine Zusammensetzung, mit der man eine für medizinische Zwecke ausreichende Wirkung erzielen kann. Die gemahlenen Rohdrogen sowie ethanol-wässrige Auszüge bzw. daraus hergestellte Trockenauszüge (herstellbar z.B. durch Abziehen des Lösungs-/Extraktionsmittels unter vermindertem Druck) der oben genannten Pflanzen haben sich durch ihre ausschließlich auf pflanzlicher Basis beruhenden Heilkraft bewährt. Die in Imupret® verwendeten Heilpflanzen werden sorgfältig ausgewählt, untersucht und weiterverarbeitet. Die gleichbleibende Qualität des Arzneimittels erreicht BIONORICA durch optimal erarbeitete Anzucht-und Erntestrategien und strengste Qualitätskontrolle.

Jede Einzeldroge trägt dabei einen Anteil zur einzigartigen Wirksamkeit der Zusammensetzung bei. Diese Zusammensetzung ist besonders geeignet und beschrieben für die Behandlung von Atemwegsinfektionen, insbesondere rezidivierende und chronische Atemwegsinfekte, vorzugsweise Tonsillitis. Bei der Tonsillitis oder synonym Angina, (Tonsillo)Pharyngitis, sog. Mandelentzündung besteht eine Entzündung der lymphoepithelialen Gewebe des lymphatischen Rachenrings, insbesondere der Gaumenmandeln. Man unterscheidet zwischen a.) Tonsillitis acuta: wird meist durch Viren (ca. 80 %; Adeno-, Parainfluenzaviren) und betahämolysierende Streptokokken der Gruppe A, seltener durch Staphylo- und Pneumokokken verursacht. Die Entzündung verläuft meist mit hohem Fieber, Halsschmerzen, kloßige Sprache, Druckschmerzhaftigkeit und Schwellung der submandibulären Lymphknoten, dabei Rötung und Schwellung der Tonsillen (Angina catarrhalis), häufig einzelne Beläge (sog. Stippchen) an den Kryptenmündungen (Angina lacunaris) oder über Lymphfollikeln (Angina follicularis, Pneumokokkenangina);
und b.) Tonsillitis *chronica*: wird meist durch eine Mischinfektion mit anaeroben und aeroben Erregern unter Beteiligung betahämolysierender Streptokokken der Gruppe A (durch verschiedene Serotypen eigentlich Neuinfektionen) verursacht; pathololgisch-anatomische Retention von Zelldetritus, Kryptenabszesse, Fibrosierung und Nekrose des Parenchyms; Beteiligung des peritonsillären Gewebes. Der Entzündungsverlauf ist häufig rezidiv. Anginen; geringe Beschwerden (sog. Halskratzen), vergrößerte submandibuläre Lymphknoten, Foetor ex ore, dabei gerötete Tonsillen mit narbiger und zerklüfteter Oberfläche, peritonsillärer Druckschmerz, bei Spateldruck auf den vorderen Gaumenbogen Entleerung von Eiter und Zelldetritus aus den Krypten.

Beschrieben ist im Stand der Technik, der entzündungshemmende Effekt der Imupret® Pflanzenextrakt Kombination infolge einer Infektionskrankheit und zugehörigem Krankheitserreger.

Es besteht jedoch ein hohes Bedürfnis die Wirkung einer Imupret®-Zusammensetzung weiter zu entwickeln, insbesondere für weitere Indikationen bereitzustellen.

Die Prostaglandinbiosynthese wird durch die Umwandlung von Arachidonsäure zu Prostaglandin(PG)H₂ mittels Cyclooxygenase (COX) COX-1 oder COX-2 eingeleitet. Bestimmte Prostaglandine, wozu auch das PGE₂ gehört, sind so genannte Mediatorstoffe bei Entzündungen, insbesondere bei rheumatoider Arthritis und bei Schmerzen und Fieber. Andere Prostaglandine wiederum erfüllen relevante physiologische Funktionen.

Akute und chronische entzündliche Erkrankungen gehen mit einer erhöhten Aktivität von entzündungsrelevanten Zellen wie z.B. Monozyten, Granulozyten, Lymphozyten und endothelialen Zellen einher, die vermehrt PGE₂ und/oder 5-LOX (5-Lipoxygenase) Produkte (hauptsächlich Leukotriene (LTs)) freisetzen. Diese 5-LOX Produkte und das PGE₂ sind maßgeblich für die Entstehung und Aufrechterhaltung entzündlicher Symptomatik (Schmerz, Schwellung, Rötung, Überwärmung und Funktionsverlust) verantwortlich bzw. wirken fördernd darauf ein. Daneben fördern 5-LOX Produkte (allergisches) Asthma und allergische Rhinitis, sind an der Entstehung und Progression der Arteriosklerose beteiligt und spielen eine wichtige Rolle bei der Pathologie rheumatoider Erkrankungen, als auch COPD (Chronic Obstructive Pulmonary Disease). Weiterhin haben PGE₂ und 5-LOX Produkte eine wichtige Funktion bei der Proliferation verschiedener Zellen und sind für die Entstehung und Aufrechterhaltung von Krebserkrankungen (insb. Lungenkrebs, Darmkrebs und Krebs des Endometriums) verantwortlich.

Diese entzündungsinduzierte Zellaktivierungen verursachen eine Aktivierung von Transkriptionsfaktoren (NF-kappa B (NF-kB1), NF-IL-6, STAT) gefolgt von einer signifikanten Hochregulation proinflammatorischer Zytokine (TNF alpha, IL-6, iNOS, COX-2) innerhalb des Gewebes. Durch die vermehrte Freisetzung proinflammatorischer Zytokine kommt es zu einer Freisetzung kinetisch wirksamer Mediatoren wie NO (Stickstoffmonoxid) und Prostaglandinen.

Zu den einzelnen Pflanzenextrakten sind folgende in der Literatur für Entzündungserkrankungen beschrieben:
Kamille:
   Srivastava JK, Pandey M, Gupta S. - Life Sci. 2009 Nov 4;85(19-20):663-9. Epub 2009 Sep 27. Chamomile, a novel and selective COX-2 inhibitor with anti-inflammatory activity.
Löwenzahn:
   Jeon HJ, Kang HJ, Jung HJ, Kang YS, Lim CJ, Kim YM, Park EH - J Ethnopharmacol. 2008 Jan 4;115(1):82-8. Epub 2007 Sep 15. Anti-inflammatory activity of Taraxacum officinale.

Ausgehend vom nächsten Stand der Technik der bekannten Imupret®-Zusammensetzung ist es somit Aufgabe der vorliegenden Erfindung, weitere Arzneimittel auf der Basis dieser Pflanzenextrakte für ausgewählte Indikationen bereitzustellen.

Überraschender Weise zeigen die Extrakte aus mindestens einer Pflanzendroge *Equiseti herba* (Schachtelhalmkraut), *Juglandis folium* (Walnussblätter), *Millefolii herba* (Schafgarbenkraut), *Quercus cortex* (Eichenrinde), *Taraxaci herba* (Löwenzahnkraut), *Althaeae radix* (Eibischwurzel) und *Matricariae flos* (bzw. *Flos chamomillae* (Kamillenblüten)) eine antientzündliche Wirkung, auch für entzündliche Vorgänge, die eben nicht ursächlich auf einer Infektionserkrankung beruhen.

Besonders vorteilhaft zeigen bestimmte Kombinationen der erfindungsgemäßen Pflanzenextrakte eine verminderte Entstehung von NO (iNOS) und verminderte Ausschüttung von Transkriptionsfaktoren, wie NF-kappa B, siehe Beispiele.

Die Erfindung betrifft daher ein Arzneimittel zur Prophylaxe und Behandlung von Entzündungserkrankungen ausgewählt aus einem Pflanzenextrakt einer Mischung aus: *Juglandis folium* (Walnussblätter), *Quercus cortex* (Eichenrinde), *Matricariae flos* (bzw. *Flos chamomillae* (Kamillenblüten)).

Ein bevorzugter Pflanzenextrakt ist dadurch gekennzeichnet, dass die Extrakte mittels eines Extraktionsmittels aus 40 - 60 Vol.-%, insbesondere 50 Vol.-% Ethanol und 40 - 60 Vol.-%, insbesondere 50 Vol.-% Wasser aus dem Pflanzenmaterial über mindestens 24h unter Rühren und anschließendem Vakuumverdampfen des Lösungsmittels herstellbar sind, siehe Beispiele.

Es hat sich völlig überraschend herausgestellt, dass der erfindungsgemäße Pflanzenextrakt eine effektive antientzündliche Wirkung aufweist, und zwar vorzugsweise zur Behandlung von PGE₂- und / oder 5-LOX-vermittelter Erkrankungen oder krankhafter Zustände, insbesondere chronische Entzündungen vorzugsweise ausgewählt aus der Gruppe rheumatoide Arthritis, Osteoarthritis, Asthma, COPD (Chronic Obstructive Pulmonary Disease), allergische Rhinitis, entzündliche Hauterkrankungen, insbesondere atopische Dermatitis, Psoriasis, Osteoporose und Krebs, insbesondere Lungenkrebs, oder krankhafte Zustände, insbesondere Schmerz und Fieber.

In vorteilhafter und an sich bekannter Weise können die Pflanzenextrakte der vorliegenden Erfindung zur Herstellung eines antientzündlichen Mittels verwendet werden. Derartige antientzündliche Mittel können zur Prophylaxe und/oder zur Behandlung von Entzündungserkrankungen oral oder topisch z.B. auf Haut und Schleimhäuten in Form einer ihrer Anwendung entsprechenden galenischen Formulierung eingesetzt werden.

Die galenischen Formulierungen der antientzündlichen Mittel der vorliegenden Erfindung sind dadurch gekennzeichnet, dass die oralen Formulierungen wie Dragees, Tabletten, Filmtabletten, Pulver, Kapseln oder flüssige Verdünnungen, insbesondere Tropfen, Säfte oder Sirupe umfassen.

Bei Einsatz zur topischen Applikation eignen sich insbesondere Sprays, Salben, Emulsionen, Puder, Pulver, flüssige oder feste Präparate für die Inhalation, Kompressen, Wund- und Zahnfleischeinlagen, Tamponaden, Tonsillenpinsellösungen, Gurgellösungen oder Spüllösungen für Nase und Ohr. Dabei sind die oben angesprochenen Tamponaden auch für zahnärztliche Anwendungen geeignet.

In einer weiteren Ausführung der Erfindung können die wäßrig/alkoholischen Pflanzenextrakte in eine Trockenmasse überführt werden. In einer bevorzugten Ausführungsform wird eine Gefriertrocknung der Pflanzenextrakte durchgeführt. Andere Trocknungsverfahren sind jedoch ebenfalls einsetzbar.

Sowohl die erfindungsgemäßen Pflanzenextrakte als auch deren wäßrige Suspensionen und Trockenmassen zeigen eine antientzündliche Wirkung.

Die Erfindung betrifft ebenfalls ein Arzneimittel, bestehend aus einem erfindungsgemäßen Pflanzenextrakt der genannten Pflanzen(drogen), wie oben beschrieben, oder Trockenmasse oder einem erfindungsgemäßen antientzündlichen Mittel zur Behandlung von chronischen Entzündungserkrankungen oder die Verwendung der erfindungsgemäßen antientzündlichen Mittel als Arzneimittel.

Ferner betrifft die Erfindung eine pharmazeutische Formulierung enthaltend ein erfindungsgemäßes Arzneimittel bzw. antientzündliches Mittel.
Die antientzündlichen Mittel können in Form von pharmazeutischen Zubereitungen in Dosierungseinheiten zubereitet werden. Dies bedeutet, dass die Zubereitung in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben, einem Drittel oder einem Viertel einer Tagesdosis entspricht.
Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Formulierungen seien Tabletten, Dragees, Kapseln, Pillen, Granulate, Suppositorien, Lösungen, Saft, Suspensionen und Emulsionen, Pasten, Salben, Gele, Cremes, Lotions, Puder und (Nasen)Sprays genannt. Tabletten, Dragees, Kapseln, Pillen und Granulate können den oder die Wirkstoffe neben den üblichen Trägerstoffen enthalten, wie a) Füll- und Streckmittel, z.B. Stärken, Milchzucker, Rohrzucker, Glukose, Mannit und Kieselsäure, b) Bindemittel, z.B. Carboxymethylcellulose, Alginate, Gelatine, Polyvinylpyrrolidon, c) Feuchthaltemittel, z.B. Glycerin, d) Sprengmittel, z.B. Agar-Agar, Calciumcarbonat und Natriumcarbonat, e) Lösungsverzögerer, z.B. Paraffin und f) Resorptionsbeschleuniger, z.B. quartäre Ammoniumverbindungen, g) Netzmittel, z.B. Cetylalkohol, Glycerinmonostearat, h) Adsorptionsmittel, z.B. Kaolin und Bentonit und i) Gleitmittel, z.B. Talkum, Calcium- und Magnesiumstearat und feste Polyethylenglykole oder Gemische der unter a) bis i) aufgeführten Stoffe.
Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können. Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. C14-Alkohol mit C16-Fettsäure) oder Gemische dieser Stoffe.
Salben, Pasten, Cremes und Gele können neben dem oder den Wirkstoffen die üblichen Trägerstoffe enthalten, z.B. tierische und pflanzliche Fette, Wachse, Paraffine, Stärke, Tragant- Cellulosederivate, Polyethylenglykole, Silikone, Bentonite, Kieselsäure, Talkum und Zinkoxid oder Gemische dieser Stoffe.
Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel enthalten.
Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3- Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten. Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin, enthalten.

Weiterhin können die erfindungsgemäßen Pflanzenextrakte Gegenstand einer dermatologischen Zusammensetzung, wie oben ausgeführt oder ein Kosmetikum sein.

Daher betrifft die Erfindung ebenfalls ein Kosmetikum oder dermatologische Zusammensetzung oder ein Mittel zur Körper- und Schönheitspflege enthaltend ein antientzündliches Mittel gemäß der vorliegenden Erfindung.

In einer weiteren Ausführungsform kann die erfindungsgemäße kosmetische oder dermatologische Zubereitung zur topischen Verwendung in Form einer Salbe, Creme, Gel, Lotion (Hautmilch), Paste oder vorzugsweise Emulsion erfolgen. Ebenfalls sind wasserfreie Systeme möglich.

Zu den erfindungsgemäß geeigneten wasserfreien Systemen gehören reine Ölzubereitungen wie z.B. Hautöle. Ebenfalls einsetzbare Pasten enthaltend die erfindungsgemäße Zubereitung, zeichnen sich dadurch aus, das sie aus den gleichen oder ähnlichen Bestandteilen wie eine Emulsion bestehen, jedoch im wesentlichen wasserfrei sind. In einer weiteren bevorzugten Ausführungsform kann die erfindungsgemäße Zubereitung als weiteren Bestandteil einen Emulgator beinhalten. In einer ganz bevorzugten Ausführung kann dieser Emulgator ein O/W-Emulgator sein.

Zur Herstellung der erfindungsgemäßen Pflanzenextrakte und deren Kombinationen aus den Pflanzen wird auf die technischen Lehren, die Gegenstand von EP 1368605B1, EP 0753306B1 sind, verwiesen.

Weitere Vorteile und Merkmale der vorliegenden Erfindung ergeben sich aufgrund der Beschreibung von Ausführungsbeispielen. Die nachfolgenden Beispiele dienen zur Erläuterung der Erfindung ohne die Erfindung auf diese Beispiele zu beschränken.

### Beispiele:

### Beispiel 1:

### Herstellung von ethanolischen Pflanzenextrakten

In einem 2 l Rundkolben (lichtgeschützt) wurde 1,6 l 50 % Ethanol/Wasser (dest.) (v/v) vorgelegt und 120 g des jeweils erfindungsgemäßen Pflanzenextraktes hinzugegeben. Die Lösung wurde 24 h bei Raumtemperatur gerührt (1.000 rpm).

Nach ethanolischer Extraktion wurde abfiltriert (lichtgeschützt) und die Lösung unter Stickstoffatmosphäre in der Kälte gelagert (4-8 Grad C). Die Extraktlösung wurde aufkonzentriert und im Vakuum getrocknet und anschließend eingefroren (- 20 Grad C) und lyophilisiert (Vakuum 0,05 - 0,1 mbar, min. 24 h).

### Beispiel 2:

### Hemmung der NFkB1 Genexpression; Wirkung der Pflanzenextrakte auf NFkB1

### Methode:

Eine humane Makrophagen-Zelllinie wird verwendet. 5x10E5 THP-1 Zellen/ml wurden mit Vitamin D₃ (10 ng/ml) für 2 Tage inkubiert und mit den jeweils erfindungsgemäßen Pflanzenextrakten für 1 h versetzt (Endkonzentration 50 µg/ml) und anschließend 3 h mit LPS (100 ng/ml) stimuliert. Zellen mit 0,1% DMSO dienten als Referenzzellen. Als Positivkontrolle wurde Parthenolid (15 µM) verwendet.

Total-RNA wurde mittels GenElute™ Mammalian Total RNA Kit (Sigma, USA) extrahiert, Reverse Transkription zu cDNA erfolgte mittels High Capacity cDNA Archive Kit (Applied Biosystems, USA).

Die Primer und Probennachweise für NF-kBl wurden mittels Primer Express 2.0.0 software (Applied Biosystems, USA) durchgeführt. Die Primer und Probennachweise für GAP-DH erfolgten mit PreDeveloped TaqMan® Assay VIC-MGB, Applied Biosystems.

Die Expression von NF-kB1 standardisiert auf GAP-DH wurde mit SDS 1.2.3. software (Applied Biosystems, USA) durchgeführt ("comparative Ct method of quantification").

### Hemmung der COX 2 Expression; Wirkung der Pflanzenextrakte auf COX-2

### Methode:

2 x 105 THP-1 Zellen/ml wurden mit Phorbol-12-myristat-13-acetat (10 ng/ml) für 2 days inkubiert. und mit den jeweils erfindungsgemäßen Pflanzenextrakten für 1 h versetzt (Endkonzentration 50 µg/ml) und anschließend 1 h mit LPS (100 ng/ml) stimuliert Zellen mit 0,1% DMSO dienten als Referenzzellen. Als Positivkontrolle wurde Dexamethason (500 nM) verwendet.

Die Primer und Probennachweise für COX-2 wurden mittels Primer Express 2.0.0 software (Applied Biosystems, USA) durchgeführt. Die Primer und Probennachweise für GAP-DH erfolgten mit PreDeveloped TaqMan® Assay VIC-MGB, Applied Biosystems.

Die Expression von COX-2 standardisiert auf GAP-DH wurde mit SDS 1.2.3. software (Applied Biosystems, USA) durchgeführt ("comparative Ct method of quantification").

**Tabelle 1: Inhibition von NFkB1 und COX-2 expression in %**

| | **Positivkontrolle** | **Quercus cortex** | **Altheae herba** | **Matricariae flos** | **Taraxaci herba** |
|---|---|---|---|---|---|
| NFkB1 expr. | 92.21 | 33.43 | -17.39 | -8.36 | 10.98 |
| COX-2 expr. | 83.84 | 85.26 | 49.27 | 64.11 | 17.18 |

**Tabelle 2: Inhibition von NFkB1 und COX-2 expression in %**

| | **Positivkontrolle** | **Juglandis folium** | **Millefolii herba** | **Equiseti herba** | **Imupret®** |
|---|---|---|---|---|---|
| NFkB1 expr. | 92.21 | 18.57 | 9.31 | -3.28 | 13.61 |
| COX-2 expr. | 83.84 | 68.40 | 45.99 | 45.36 | 56.51 |

### xx.xx Inhibition der Pflanzenextrakte ≥ 50% Inhibition der Positivkontrolle

### Beispiel 3:

### Hemmung des Arachidonsäure-Pathway (COX-1, COX-2, und 5-LOX); Wirkung der Pflanzenextrakte auf COX-1, COX-2 und 5-LOX Hemmung als auch Hemmung der NO-Entstehung in vitro

### Methode:

### Analyse der Hemmung auf die Enzyme COX-1 und COX-2

Test kit: Assay Designs: PGE2 Correlate EIA Kit (Cat. No. 900-001)
Enzyme: Cayman Chemical Company:
   COX-1 (Schaf) aus ram seminal vesicles
   COX-2 aus sheep placental cotyledons

### Durchführung:

Die Durchführung erfolgte gemäß dem Test Kit PGE2 Correlate EIA - Kit-Protokoll, das jeweilige Enzym (0.2 U/well) wurde in Puffer inkubiert, und mit den Pflanzenextrakten und Arachidonsäure (5 µM, Cayman Chemical Company) bei 37°C in 96 well Mikrotiterplatten versetzt. Die Endkonzentration der Pflanzenextrakte betrug 50 µg/ml. Als Positivkontrolle diente Indomethacin (Endkonzentration im Assay: 1.25µM) für COX-1 und NS-398 (Endkonzentration im Assay: 5 µM) für COX-2.

Die PGE2 Entstehung wurde mit einem ELISA (405 nm) nach Herstellerangaben quantifiziert und auf COX-1 und COX-2 zurückgerechnet.

### Analyse der Hemmung auf das Enzym 5-LOX

Test kit: Assay Designs: LTB4 Correlate EIA Kit
Enzyme: stimulierte humane polymorphkerniger Leukozyten mit 5-LOX Aktivität

### Durchführung:

Die Zellsuspension wurde mit Pflanzenextrakten und Arachidonsäure (4.43 µM, Cayman Chemical Company) bei 37°C in 96 well Mikrotiterplatten versetzt. Die Endkonzentration der Pflanzenextrakte betrug 50 µg/ml. Als Positivkontrolle diente Zileuton (Endkonzentration im Assay: 10 µM).

Die LTB4 Entstehung wurde mit einem ELISA (405 nm) nach Herstellerangaben quantifiziert und auf 5-LOX zurückgerechnet.

### Analyse der Hemmung auf das induzierbare Enzym NO Synthase (iNOS)

### Durchführung:

Eingesetzte Zelllinie im Assay: RAW 264.7 Macrophagen
Die Zellen wurden mit 0.5 µg/ml LPS und 50 U/ml Interferon in An- oder Abwesenheit der Pflanzenextrakte versetzt. Der iNOS Effekt wurde mittels Messung der Nitrit-Freisetzung photometrisch bestimmt.

Als Positivkontrolle wurde N_{G}-Monomethyl-L-Arginin-Monoacetat (100 µM) verwendet.

**Tabelle 3: Inhibition von COX-1, COX-2 und 5-LOX und NO-Entstehung in %**

| | **Positivkontrolle** | **Quercus cortex** | **Altheae herba** | **Matricariae flos** | **Taraxaci herba** |
|---|---|---|---|---|---|
| COX-1 | 68.24 | 41.61 | 8.51 | -20.82 | -11.99 |
| COX-2 | 39.33 | 22.23 | 19.36 | 21.58 | 27.92 |
| 5-LOX | 73.76 | 51.18 | 38.14 | 38.51 | 40.89 |
| NO-Entstehung | 40.20 | 9.15 | 3.48 | 5.78 | 3.60 |

**Tabelle 4: Inhibition von COX-1, COX-2 und 5-LOX und NO-Entstehung in %**

| | **Positivkontrolle** | **Juglandis folium** | **Millefolii herba** | **Equiseti herba** | **Imupret** |
|---|---|---|---|---|---|
| COX-1 | 68.24 | 14.18 | -14.27 | -1.07 | 23.14 |
| COX-2 | 39.33 | 12.41 | 20.86 | 32.94 | 31.26 |
| 5-LOX | 73.76 | 39.14 | 37.00 | 22.94 | 28.61 |
| NO-Entstehung | 40.20 | 5.20 | 6.27 | 1.82 | 1.92 |

xx.xx Inhibition der Pflanzenextrakte ≥ 50% Inhibition der Positivkontrolle

## Patentansprüche

1. Arzneimittel zur Verwendung in der Prophylaxe und Behandlung von chronischen Entzündungserkrankungen ausgewählt aus einem Pflanzenextrakt einer Mischung aus: *Juglandis folium* (Walnussblätter), *Quercus cortex* (Eichenrinde), *Matricariae flos* (bzw. *Flos chamomillae* (Kamillenblüten)).

2. Arzneimittel zur Verwendung nach Anspruch 1, **dadurch gekennzeichnet, dass** die Pflanzenextrakte mittels eines Extraktionsmittels aus 40 - 60 Vol. -% Ethanol und 40 - 60 Vol. -% Wasser aus dem Pflanzenmaterial über 6 bis 36 h, insbesondere 12 bis 30 h, unter Rühren und ggfs. Vakuumverdampfen des Lösungsmittel herstellbar sind.

3. Arzneimittel zur Verwendung nach einem der vorhergehenden Ansprüche, zur Verwendung in der Prophylaxe und Behandlung von PGE₂- und / oder 5-LOX-vermittelter Erkrankungen oder krankhafter Zustände.

4. Arzneimittel zur Verwendung nach einem der vorhergehenden Ansprüche, zur Verwendung in der Prophylaxe und Behandlung von chronischen Entzündungen ausgewählt aus der Gruppe rheumatoide Arthritis, Osteoarthritis, Asthma, COPD (Chronic Obstructive Pulmonary Disease), allergische Rhinitis, entzündliche Hauterkrankungen, insbesondere atopische Dermatitis, Psoriasis, Osteoporose und Krebs, insbesondere Lungenkrebs, oder krankhafte Zustände, insbesondere Schmerz und Fieber.

5. Arzneimittel zur Verwendung nach einem der vorhergehenden Ansprüche, **dadurch gekennzeichnet, dass** es in einer galenischen Formulierung vorliegt, insbesondere eine orale Formulierung wie Dragees, Tabletten, Filmtabletten, Kapseln, flüssige Verdünnungen, insbesondere Tropfen, Säfte, Sirupe oder eine Formulierung zur topischen Anwendung wie Sprays, Salben, Emulsionen, Puder, Pulver, flüssige oder feste Präparate für die Inhalation, Kompressen, Wund-und Zahnfleischeinlagen, Tamponaden, Tonsillenpinsellösungen, Gurgellösungen oder Spüllösungen für Nase und Ohr oder Lyophilisat.

## Claims

1. A pharmaceutical for use in the prophylaxis and the treatment of chronic inflammatory diseases, selected from a plant extract of a mixture of: *Juglandis folium* (walnut leaf), *Quercus cortex* (oak bark), *Matricariae flos* (or *Flos chamomillae* (chamomile flower)).

2. The pharmaceutical for use according to claim 1, **characterized in that** the plant extracts can be produced from the plant material using an extracting agent comprising 40 to 60% by volume ethanol and 40 to 60% by volume water over 6 hours to 36 hours, and more particularly 12 to 30 hours, while stirring and optionally vacuum evaporating the solvent.

3. The pharmaceutical for use according to any one of the preceding claims, for use in the prophylaxis and the treatment of diseases or morbid conditions mediated by PGE₂ and/or 5-LOX.

4. A pharmaceutical for use according to any one of the preceding claims, for use in the prophylaxis and the treatment of chronic inflammatory diseases selected from the group consisting of rheumatoid arthritis, osteoarthritis, asthma, chronic obstructive pulmonary disease (COPOD), allergic rhinitis, inflammatory skin diseases, in particular atopic dermatitis, psoriasis, osteoporosis, and cancer, in particular lung cancer, or morbid conditions, in particular pain and fever.

5. A pharmaceutical for use according to any one of the preceding claims, **characterized by** being present in a galenic formulation, in particular in an oral formulation such as coated tablets, tablets, film-coated tablets, capsules, liquid dilutions, in particular drops, juices, syrups, or a formulation for topical application such as sprays, ointments, emulsions, fine powders, coarse powders, liquid or solid preparations for inhalation, compresses, wound and gum dressings, tamponades, tonsil brush solutions, gargling solutions or rinsing solutions for the nose and ears, or a lyophilizate.

## Revendications

1. Produit pharmaceutique pour une utilisation dans la prophylaxie et le traitement de maladies inflammatoires chroniques choisi à partir d'un extrait de plantes d'un mélange constitué de : *Juglandis folium* (feuilles de noyer), *Quercus cortex* (écorce de chêne), *Matricariae flos* (respectivement, *Flos chamomillae* (fleur de camomille)).

2. Produit pharmaceutique pour une utilisation selon la revendication 1, **caractérisé en ce que** les extraits de plantes peuvent être préparés au moyen d'un produit d'extraction constitué de 40 à 60 % en volume d'éthanol et de 40 à 60 % en volume d'eau à partir de la matière végétale pendant 6 à 36 heures, notamment pendant 12 à 30 heures, moyennant une agitation et éventuellement une évaporation sous vide du solvant.

3. Produit pharmaceutique pour une utilisation selon l'une des revendications précédentes, pour une utilisation dans la prophylaxie et le traitement de maladies à médiation par la PGE₂ et/ou la 5-LOX ou d'états maladifs.

4. Produit pharmaceutique pour une utilisation selon l'une des revendications précédentes, pour l'utilisation dans la prophylaxie et le traitement d'inflammations chroniques choisies dans le groupe constitué de l'arthrite rhumatoïde, de l'ostéoarthrite, de l'asthme, de la COPD (maladie pulmonaire obstructive chronique), de la rhinite allergique, de maladies de la peau inflammatoires, notamment, de la dermatite atopique, du psoriasis, de l'ostéoporose et du cancer, notamment, le cancer du poumon, ou d'états maladifs, notamment de douleurs et de fièvre.

5. Produit pharmaceutique pour une utilisation selon l'une des revendications précédentes, **caractérisé en ce qu'**il est présent dans une formulation galénique, notamment une formulation orale comme des comprimés, des pastilles, des comprimés pelliculés, des gélules, des dilutions liquides, notamment, des gouttes, des jus, des sirops, ou une formulation pour une utilisation topique comme des sprays, des pommades, des émulsions, de la poudre, des produits pulvérulents, des préparations liquides ou solides pour l'inhalation, des compresses, des pansements pour les plaies et les gencives, des tamponnades, des solutions à badigeonner les amygdales, des solutions pour des gargarismes ou des solutions de lavage pour le nez et les oreilles ou de lyophilisat.
